# EUROPÄISCHE PATENTANMELDUNG

(11) **EP 1 080 700 A2**
(43) Veröffentlichungstag der Anmeldung: **07.03.2001**
(21) Anmeldenummer: 00118539.6
(22) Anmeldetag: 25.08.2000
(51) Int. Cl.: A61F 2/28

(54) **Folie aus resorbierbarem Polymermaterial und Verfahren zur Herstellung einer solchen Folie**

(30) Priorität: 28.08.1999 DE 19940977
(71) Anmelder: Claes, Lutz, Prof.-Dr., 89233 Neu-Ulm/Pfuhl (DE)
(72) Erfinder: Claes, Lutz, Prof.-Dr., 89233 Neu-Ulm/Pfuhl (DE); Kreidler, Joachim, Prof.-Dr., 89143 Blaubeuren-Seissen (DE); Ignatius, Anita, Dr., 89275 Elchingen (DE); Palm, Frank, Dr., 89075 Ulm (DE)
(74) Vertreter: Weber, Gerhard

(57) **Zusammenfassung**

Für die Abdeckung eines Knochendefekts in Form einer Knochenhöhlung, insbesondere im Bereich der Kieferchirurgie wird eine Folie vorgeschlagen, deren Eigenschaften hinsichtlich mechanischer Stabilität und Degradation besonders vorteilhaft für den Heilungsprozeß des Knochendefekts sind. Besonders vorteilhaft ist eine Folie aus resorbierbarem Material, welche auf Ihrer der Knochenhöhlung zugewandten Seite mit einem osteokonduktiven Material beschichtet ist.

## Beschreibung

Die Erfindung betrifft ein Folie aus resorbierbarem Polymermaterial sowie ein Verfahren zur Herstellung einer solchen Folie.

Zur Ausheilung von Knochendefekten in Form von Höhlungen oder Ausnehmungen in Knochen ist das Prinzip der sogenannten Guided Bone Regeneration bekannt, welches beispielsweise nach einer Zahnextraktion das Zuwachsen einer Höhlung im Kieferknochen mit Knochenmaterial ermöglicht. Hierbei werden Folien oder Membranen über die Defektstellen gelegt, um als Barriere gegen das Eindringen von Fibroblasten und Gingivazellen in die Höhlung zu wirken, so dass die gesamte Höhlung mit Knochenmaterial zuwachsen kann.

Besonders häufig eingesetzt werden hierzu Membranen aus PTFE. Nachteilig daran ist, daß diese Membranen nach dem Heilungsprozeß wieder entfernt werden müssen, wofür das überdeckende Weichgewebe wieder aufgeschnitten werden muß mit allen unangenehmen Begleiterscheinungen für den Patienten. Das Belassen der PTFE-Membranen im Körper kann zu Komplikationen Anlaß geben.

In der EP 0 609 667 A1 ist zur Beseitigung dieser Nachteile eine Abdeckmembran beschrieben, welche aus resorbierbarem Material besteht. Solches Material wird im Körper zu unschädlichen Produkten abgebaut und abtransportiert und löst sich so über einen längeren Zeitraum auf. Die Membran kann dabei Materialien wie lyophilisierte Hirnhaut, Polylactid oder vorzugsweise Kollagen enthalten.

Resorbierbare Materialien sind auch in anderen Bereichen der Chirurgie als körperabbaubare Hilfsmittel eingesetzt. Beispielsweise sind aus der EP 0 401 844 B1 Formkörper aus resorbierbarem Material als chirurgische Implantate, als Fraktur-Stützmittel, aber auch als Gefäßverschlußklipps usw. bekannt. Als Material sind Polylactid oder davon abgeleitete Copolymerisate mit einem hochmolekularen Zusatzstoff aus einem resorbierbarem Polyester vorgesehen. Die Formkörper werden bevorzugt im Spritzgießverfahren hergestellt.

Bei der Ausheilung von Knochendefekten im Kieferknochen ist besonders zu beachten, daß durch die starke Durchblutung der überdeckenden Schleimhaut die Degradation resorbierbaren Membranmaterials begünstigt wird, und dass durch bewußte oder unbewußte Zungenbewegungen oder Kauvorgängen über der Defektstelle die Abdeckmembran besonderer mechanischer Belastung ausgesetzt ist.

In der genannten EP 0 609 667 A1 ist die Knochenhöhlung mit Knochenaufbaumaterial vorzugsweise einem Granulat aus Hydroxilapatit ausgefüllt, so daß die Abdeckmembran von innen stabil abgestütz ist. Es sind auch Abdeckmembranen mit Versteifungselementen bekannt.

Der Erfindung liegt die Aufgabe zugrunde, eine Folie zur Abdeckung von Knochenhöhlungen anzugeben, welche besonders vorteilhaft für den Ausheilungsprozeß ist, sowie ein Verfahren zur Herstellung einer solchen Folie.

Erfindungsgemäße Lösungen sind in den unabhängigen Ansprüchen beschrieben. Die abhängigen Ansprüche enthalten vorteilhafte Ausgestaltungen und Weiterbildungen der Erfindung.

Durch die anfänglich über einen Zeitraum von wenigstens 7 Wochen geringe Abnahme der mechanischen Stabilität der Folie, welche insbesondere durch die Zugfestigkeit des Polymermaterials ausgedrückt ist, ist eine zuverlässige Abdeckung der Knochenhöhlung über einen wichtigen Zeitraum des Heilungsprozesses gewährleistet, ohne dass die Folie bleibend eingedrückt wird oder reißt. Die Abnahme der Zugfestigkeit der Folie liegt innerhalb eines Zeitraums von 7 Wochen vorteilhafterweise bei weniger als 25%, insbesondere weniger als 15% bezogen auf einen Ausgangswert der Zugfestigkeit in feuchtem Zustand zum Zeitpunkt des Einsetzens der Folie als Abdeckmembran. Die anfängliche Zugfestigkeit der Folie beträgt vorzugsweise wenigstens 20 MPa. Die Degradation des resorbierbaren Polymermaterials kann in an sich bekannter Weise in Pufferlösungen, beispielsweise einem Sörensen-Puffer nachgebildet werden.

Eine geringe anfängliche Abnahme der mechanischen Festigkeit der eingesetzten Folie erlaubt eine geringe Materialstärke für eine stabile Abdeckung, was vorteilhaft hinsichtlich der Gesamtmenge der Abbauprodukte ist. Die Dicke der Folie liegt vorzugsweise zwischen 0,05 mm und 1 mm, insbesondere zwischen 0,1 mm und 0,5 mm. Das anfängliche mittlere Molekulargewicht des Polymermaterials der Folie zum Zeitpunkt des Einsetzens als Abdeckmembrans liegt vorteilhafterweise zwischen 100 000 D und 200 000 D, wodurch zum einen eine gute anfängliche mechanische Stabilität der Folie und zum anderen eine akzeptable Resorptionszeit erreicht werden kann.

Das Folienmaterial ist vorteilhafterweise bei Körpertemperatur und den zu erwartenden mechanischen Belastungen formstabil, zugleich aber zur Anpassung an die Umgebung der Knochenhöhlung in einem Temperaturbereich zwischen 50° C und 80° C plastisch verformbar. Eine passend ausgeschnittene oder vorgeformte Folienfläche kann dann thermoplastisch 3-dimensional an die Umgebung der Knochenhöhlung optimal angepaßt werden.

Ein wichtiger Vorteil einer anfänglich geringen Abnahme der mechanischen Stabilität der Folie ist auch darin zu sehen, daß auf ein Auffüllen der Knochenhöhlung mit Knochenersatzmaterial verzichtet werden kann und somit die Knochenhöhlung vollständig mit körpereigenem Knochenmaterial zuwächst. Die Stabilität der Folie genügt während der wichtigen Knochenwachstumsphase als mechanische Abstützung gegen das konkurrierende Wachstum und den Druck umgebenden Weichgewebes.

Gemäß einer besonders vorteilhaften Variante enthält die Folie ein osteokonduktives Material, welches gleichmäßig in dem Polymermaterial der Folie verteilt sein kann und/oder als einseitige Beschichtung der Folie vorliegt, wobei beim Einsetzen der Folie die mit dem osteokonduktiven Material beschichtete Seite der Höhlung zugewandt ist. Das osteokonduktive Material liegt vorteilhafterweise als an sich bekannte körnige Keramik, vorzugsweise mit einer mittleren Korngröße von mehr als 20 µm, insbesondere mehr als 50 µm und höchstens 200 µm, insbesondere höchstens 150 µm vor und kann insbesondere Tricalciumphosphat als Wirksubstanz enthalten.

Der Einsatz des osteokonduktiven Materials, insbesondere in Form der einseitigen Beschichtung, zeigt den besonderen Vorteil, daß durch die osteokonduktive Eigenschaft neues Knochenmaterial auch in starkem Maße von den Rändern der Knochenhöhlung entlang der abdeckenden Folie wächst. Hierdurch wird zum einen das Zuwachsen der Knochenhöhlung beschleunigt, da das Wachstum von Knochensubstanz auch von Seiten der abgedeckten Öffnung der Knochenhöhlung erfolgt, und zum anderen trägt das von den Rändern der Knochenhöhlung einwachsende Knochenmaterial zur mechanischen Stabilisierung der Abdeckmembran bei und kompensiert dadurch eine eventuell durch Degradation des Polymermaterials eintretende Verringerung der mechanischen Stabilität der Abdeckmembran. Der Einsatz des osteokonduktiven Materials kann daher auch vorteilhaft für Folienmaterial sein, welches die vorstehend beschriebenen Anforderungen an die geringe Abnahme der mechanischen Stabilität nicht erfüllt und eine stärkere Degradation zeigt.

Das osteokonduktive Material zeigt zudem die vorteilhafte Eigenschaft, daß die abdeckende Folie, welche in einem die Ränder die Knochenhöhlung überragenden Bereich mit dem Knochen überlappt, in dem überlappenden Bereich eine Verankerung der Folie mit dem Knochen erreichen kann.

Das osteokonduktive Material kann dem Polymermaterial vor der Herstellung zugemischt werden oder vorzugsweise zur Erzeugung der einseitigen Beschichtung in die fertige Folie einseitig eingepreßt werden, wobei die Folie in einen Temperaturbereich plastischer Verformbarkeit gebracht werden kann.

Die Folie kann ferner zur Beschleunigung des Wachstums neuen Knochenmaterials eine Knochenwachstum fördernde osteoinduktive Substanz, z. B. sogenannte Wachstumsfaktoren, enthalten.

Die Folie kann gemäß einer vorteilhaften Ausführungsform, offenporig aufgebaut sein und dadurch den Flüssigkeitsaustausch zwischen der abgedeckten Knochenhöhlung und dem umgebenden Gewebe ermöglichen. Um zu verhindern, daß Gewebe durch die Poren und in die Höhlung einwächst, ist die Porengröße vorzugsweise kleiner als 40 µm, insbesondere kleiner als 20 µm.

Resorbierbare Polymermaterialen sind an sich in größerer Zahl bekannt, wie beispielsweise aus der genannten EP 0 401 844 B1. Die erfindungsgemäßen Eigenschaften hinsichtlich mechanischer Stabilität und/oder Resorbierbarkeit sind typischerweise Übermischungsverhältnisse von Monomeren oder Polymeren, über Zusatzstoffe und über Parameter der Herstellungsverfahren beeinflußbar und einstellbar.

Als besonders vorteilhaft erweist sich ein Polymermaterial, welches Poly(L-lactid) als Hauptbestandteil, aber nicht ausschließlich enthält. Der Anteil von Poly(L-lactid) liegt vorzugsweise höher als 65 Gew.-%, jedoch nicht höher als 95% insbesondere nicht höher als 90%. Bevorzugt ist als Polymermaterial ein Poly(L,DL-lactid), welches aus einer Polymerisation von Poly(L-lactid) und Poly(DL-lactid) erhältlich ist.

Eine Folie wird insbesondere als ebene Folie durch Anwendung eines Heißpreßverfahrens auf Polymerausgangsmaterial vorzugsweise in Form eines Granulat in der gewünschten Dicke hergestellt. Beim Heißpressen werden vorzugsweise eine Temperatur über 150°C und ein Druck über 250 bar eingesetzt. Eine Anpassung in Flächengröße und dreidimensionaler Formgebung erfolgt vorzugsweise in nachfolgenden getrennten Schritten. Die heißgepreßte Folie wird gemäß einer bevorzugten Ausführungsform einseitig mit einem körnigen osteokonduktiven Keramikmaterial beschichtet, indem dieses Keramikmaterial in die Folienfläche eingepreßt wird. Die ggf. beschichtete Folie wird vorteilhafterweise gamma-sterilisiert.

Da beim Heißpressen und/oder der gamma-Sterilisierung typischerweise eine Verringerung des mittleren Molekulargewichts des Polymermaterials eintritt, wird das Molekulargewichts des Polymerausgangsmaterials vorteilhafterweise wesentlich höher gewählt als das für die Folie vorgesehene anfängliche Molekulargewicht des Polymermaterials. Das Polymerausgangsmaterial liegt vorzugsweise als an sich gebräuchliches Granulat vor, was insbesondere für einen gleichmäßigen Einbau für körnigen osteokonduktiven Keramikmaterial besonders vorteilhaft ist.

### Beispiel:

Als Polymerausgangsmaterial diente ein Poly(L,DL-lactid) 70/30 in Granulatform. Das Rohmaterial wies ein mittleres Molekulargewicht von 670 000 D und eine inhärente Viskosität von 5,64 dl/g auf. Aus dem Granulat wurde durch Heißpressen bei 190° C und 400 bar eine homogene Folie mit einer Dicke von 0,2 mm hergestellt. Für die Beschichtung mit osteokonduktivem Material wurde ein Granulat aus α-TCP mit einer Korngröße von < 200 µm auf eine Seite der Membran aufgepreßt. Die beschichtete Membran wurde mit 25 kGy gamma-sterilisiert. Die fertige Folie zeigte ein durch das Herstellungsverfahren verringertes mittleres Molekulargewicht des Polymermaterials von 140 000 D und eine inhärente Viskosität von 1,32 dl/g. Die anfängliche Zugfestigkeit der beschichteten Folie betrug in trockenem Zustand 37,2 MPa, in feuchtem Zustand 27,2 MPa. Die Zugfestigkeit der bei 37°C in Sörensen-Puffer eingelegten Folie blieb über 8 Wochen nahezu konstant (Abnahme <5%) und fiel bis zur 20. Woche auf 6,7 MPa ab. Das Molekulargewicht erniedrigte sich bis zur 20. Woche auf 77 000 D.

Das beispielhaft angegebene bevorzugte Material zeigt sich damit hinsichtlich der mechanischen Eigenschaften und der Degradation vorzüglich geeignet. Ein weiterer Vorteil diese Materials ist, daß die Folie in auf ca. 60°C erwärmtem Zustand plastisch verformbar ist und dem Knochen anmodelliert werden kann.

Die vorstehend und in den Ansprüchen angegebenen Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

## Patentansprüche

1. Folie aus resorbierbarem Polymer-Material zum Abdecken einer Knochenhöhlung, dadurch gekennzeichnet, daß das Polymermaterial so gewählt ist, daß die Zugfestigkeit der Membran in Körperumgebung innerhalb von 7 Wochen um weniger als 25%, insbesondere um weniger als 15% abnimmt.

2. Membran nach Anspruch 1, dadurch gekennzeichnet, daß die Materialstärke des Polymermaterials zwischen 0,05 mm und 1 mm, insbesondere zwischen 0,1 mm und 0,5 mm liegt.

3. Folie aus resorbierbarem Polymer-Material zum Abdecken einer Knochenhöhlung, insbesondere nach Anspruch 1 oder 2, dadurch gekennzeichnet, daß die Folie ein osteokonduktives Material enthält.

4. Folie nach Anspruch 3, gekennzeichnet durch Tricalciumphosphat als osteokonduktives Material.

5. Folie nach Anspruch 3 oder 4, dadurch gekennzeichnet, daß das osteokonduktive Material als keramisches Material ausgebildet ist.

6. Folie nach einem der Ansprüche 3 bis 5, dadurch gekennzeichnet, daß das osteokonduktive Material in körniger Form mit mittlerer Korngröße von wenigstens 20 µm, insbesondere wenigstens 50 µm und höchstens 200 µm, insbesondere höchstens 150 µm vorliegt.

7. Folie nach einem der Ansprüche 3 bis 6, dadurch gekennzeichnet, daß die Folie einseitig auf ihrer der Knochenhöhlung zugewandten Seite mit dem osteokonduktiven Material beschichtet ist.

8. Folie nach einem der Ansprüche 3 bis 7, dadurch gekennzeichnet, daß osteokonduktives Material in das Polymermaterial eingebaut ist.

9. Folie nach einem der Ansprüche 1 bis 8, dadurch gekennzeichnet, daß die Folie eine das Knochenwachstum fördernde, osteoinduktiv wirkende Substanz enthält (z. B. Wachstumsfaktoren).

10. Folie nach einem der Ansprüche 1 bis 9, dadurch gekennzeichnet, daß die anfängliche Zugfestigkeit der Folie in feuchtem Zustand mehr als 20 MPa beträgt.

11. Membran nach einem der Ansprüche 1 bis 10, dadurch gekennzeichnet, daß das Polymermaterial Poly(L-lactid) als Hauptbestandteil enthält.

12. Folie nach Anspruch 11, dadurch gekennzeichnet, daß der Anteil von Poly(L-lactid) mehr als 50%, insbesondere mehr als 65% und höchstens 95%, insbesondere höchstens 90% beträgt.

13. Folie nach Anspruch 11, gekennzeichnet durch Poly(L,DL-lactid) als Polymermaterial.

14. Membran nach einem der Ansprüche 1 bis 13, dadurch gekennzeichnet, daß die Membran bei Temperaturen zwischen 50°C und 80°C thermoplastisch verformbar ist.

15. Folie nach einem der Ansprüche 1 bis 14, dadurch gekennzeichnet, daß das Polymermaterial Poren mit einem Porendurchmesser von weniger als 40 µm, insbesondere weniger als 20 µm aufweist.

16. Verfahren zur Herstellung einer Folie aus resorbierbarem Polymer-Material nach einem der Ansprüche 1 bis 15, gekennzeichnet durch die Anwendung eines Heißpreßverfahrens auf eine dickere Schicht eines polymeren Ausgangsmaterials.

17. Verfahren nach Anspruch 16, dadurch gekennzeichnet, daß beim Heißpressen eine Temperatur über 150°C und ein Druck über 250 bar angewandt werden.

18. Verfahren nach Anspruch 17, dadurch gekennzeichnet, daß ein Polymergranulat als Ausgangsmaterial eingesetzt wird.

19. Verfahren nach einem der Ansprüche 16 bis 18, dadurch gekennzeichnet, daß eine Schicht aus osteokonduktivem Material auf eine Seite einer Folie aus Polymermaterial aufgepreßt wird.
